# EUROPÄISCHE PATENTANMELDUNG

(11) **EP 1 741 432 A1**
(43) Veröffentlichungstag der Anmeldung: **10.01.2007**
(21) Anmeldenummer: 05014743.8
(22) Anmeldetag: 07.07.2005
(51) Int. Cl.: A61K 31/506

(54) **Tyrosinkinase-Inhibitor Imatinib zur Behandlung von Bluthochdruck**

(71) Anmelder: Universitätsklinikum Freiburg, 79106 Freiburg (DE)
(72) Erfinder: Donauer, Johannes, Dr. med., 79289 Horben (DE); Schwabe, Michael, Dr. med., 79102 Freiburg (DE)
(74) Vertreter: Keller, Günter

(57) **Zusammenfassung**

Offenbart werden Tyrosinkinase-Inhibitoren und deren Verwendung zur Herstellung eines Arzneimittels zur Behandlung von Bluthochdruck.

## Beschreibung

Die vorliegende Erfindung betrifft die Verwendung von Tyrosinkinase-Inhibitoren zur Herstellung von Arzneimitteln zur Behandlung von Bluthochdruck.

Die obere Grenze des normalen Blutdrucks beträgt nach einer Definition der WHO für den systolischen Wert 139 und für den diastolischen Wert 89 mm Quecksilbersäule. Bei dauerhaft, wenn auch nur leicht erhöhten Blutdruckwerten besteht ein erhöhtes Schlaganfall- und Herzinfarktrisiko. Für die Diagnose müssen mindestens dreimal erhöhte Blutdruckwerte bei mindestens zwei verschiedenen Gelegenheiten gemessen werden.

Die Prävalenz der arteriellen Hypertonie liegt bei der weißen Bevölkerung in Industrietändern bei ca. 25 % und steigt mit zunehmendem Alter auf über 50 % an. Nur in etwa 5 bis 10 % der Fälle kann eine organische Ursache für die erhöhten Blutdruckwerte gefunden werden und nur in diesen Fällen ist eine kausale Behandlung möglich. Bei der Mehrheit der Patienten muß diese Erkrankung als nicht heilbar angesehen werden und daher lebenslang mit blutdrucksenkenden Medikamenten behandelt werden. Diese sehr große Gruppe von Patienten wird unter dem Begriff "essentielle arterielle Hypertonie" zusammengefasst.

In Abhängigkeit vom Ausmaß der Blutdruckerhöhung wird von milder (Grad 1), moderater (Grad 2) oder schwerer (Grad 3) Hypertonie gesprochen. Die Behandlung milder Formen des Bluthochdruckes gelingt oft durch diätetische Maßnahmen, insbesondere die Verminderung des Kochsalzkonsums. Bei Patienten mit moderater oder schwerer Hypertonie müssen üblicherweise blutdrucksenkende Medikamente (sogenannte Antihypertensiva) verabreicht werden. Da bei dieser Patientengruppe üblicherweise keine eindeutige Ursache des Bluthochdrucks gefunden werden kann, stützt sich die medikamentöse Therapie auf eine Vielzahl von Medikamenten, die nach unterschiedlichen Wirkprinzipien zu einer Verminderung des erhöhten Bluthochdrucks führen.

In der Praxis wird häufig ein Patient, dessen Blutdruck nicht durch diätetische Maßnahmen allein korrigiert werden kann, mit einem Medikament eines bestimmten Wirkprinzips, z.B. einem Diuretikum, behandelt. Wenn dieses Medikament nicht ausreicht, um den Blutdruck zu normalisieren, wird häufig ein zweites Medikament verschrieben, das über einen anderen Wirkmechanismus seine Wirkung entfaltet, beispielsweise sogenannte ACE-Hemmer.

Wenn diese Medikamente zu keiner effektiven Regulierung des Blutdrucks führen, kann ein weiteres Medikament, das über einen anderen Wirkmechanismus seine Wirkung entfaltet, beispielsweise sogenannte β-Blocker, verabreicht werden.

Die medikamentöse Behandlung führt nur in einem geringeren Teil der Fälle (zwischen 30 und 40 %) zu einer ausreichenden Blutdrucksenkung. Die arterielle Hypertonie bleibt daher auch weiterhin ein bedeutender Risikofaktor für das Auftreten von Schlaganfällen und Herzinfarkten in der Bevölkerung. Gerade für diejenige Patientengruppe, die nur schlecht auf bereits bekannte blutdrucksenkende Mittel anspricht, besteht ein großes Bedürfnis nach wirksamen Arzneimitteln, die den Blutdruck wirksam senken.

Im Rahmen der vorliegenden Erfindung wurde überraschenderweise gefunden, dass sogenannte Tyrosinkinase-Inhibitoren eine blutdrucksenkende Wirkung aufweisen.

Tyrosinkinasen phosphorilieren die Aminosäure Tyrosin und spielen nach derzeitigem Kenntnisstand eine entscheidende Rolle bei der Regulierung von Zellteilung und damit auch für das Wachstum von Zellen. Gerade bei malignen Entartungen von Zellen, die bei tumorartigen Erkrankungen auftreten, können Tyrosinkinase-Inhibitoren eine vorteilhafte Wirkung entfalten.

Derzeit sind im wesentlichen zwei Tyrosinkinase-Inhibitoren auf dem Markt. Hierbei handelt es sich einerseits um ein Produkt mit dem INN "Gefitinib". Dahinter verbirgt sich die chemische Verbindung N-(3-chlor-4-fluorphenyl)-7-methoxy-6-(3-morpholinopropoxy)quinazolin-4-amin. Dieses Produkt wird derzeit von der Firma AstraZeneca unter der Marke Iressa® vertrieben. Gefitinib ist ein selektiver Inhibitor, der "epidermal growth factor receptor (EGFR)"-Tyrosinkinase.

Da die EGFR-Tyrosinkinase in den Zellen von bestimmten Typen von menschlichen Karzinomen, beispielsweise bei Lungen- und Brustkrebs überexprimiert wird, wird Gefitinib gegenwärtig für die Behandlung von bestimmten Formen von Lungenkrebs eingesetzt.

Bevorzugt wird im Rahmen der vorliegenden Erfindung ein Wirkstoff eingesetzt mit dem INN Imatinib. Hierbei handelt es sich um 4[(4-Methyl-1-piperazinyl)methyl]-N-[4-methyl-3-[[4-(3-pyridinyl)-2-pyrimidinyl]amino]-phenyl]benzamid-methansulfonat. In besonders bevorzugter Form wird das Mesylatsalz dieser Verbindung eingesetzt, grundsätzlich können aber auch andere Salze verwendet werden.

lmatinib ist ein 2-Phenylaminopyrimidin-Derivat, das als spezifischer Inhibitor bei einer Anzahl von Tyrosinkinasen wirkt. So wird Imatinib insbesondere bei der chronischen myeloischen Leukämie (CML) eingesetzt, um die Aktivität des Fusionsproteins bdr/abl zu reduzieren. Da diese Tyrosinkinase ein Molekül ATP benötigt, um die Tyrosinreste auf dem Substrat zu phosphyrilieren, bindet Imatinib anstelle dessen an diese Stelle und inhibiert das Protein vollständig.

Die chronisch-myeloische Leukämie (CML) gehört zu den myeloproliferativen Erkrankungen. CML ist eine Erkrankung, die üblicherweise leicht diagnostiziert werden kann, weil die leukämischen Zellen von mehr als 95 % der daran leidenden Patienten eine chromosomale Abnormalität, das sogenannte Philadelphia-Chromosom (Ph1) aufweisen. Das Philadelphia-Chromosom rührt von einer reziproken Translokalisation zwischen den langen Armen der Chromosomen 9 und 22 her und kann in allen hämatopoetischen Vorläuferzellen nachgewiesen werden. Diese chromosomale Umorganisation führt zur Fusionierung des bcr-Proteins mit der abl-Tyrosinkinase zu einer enzymatisch permanent aktiven Tyrosinkinase bcr/abl. Dieses Fusionsprodukt bewirkt eine unkontrollierte Vermehrung myeloischer Blutzellen und die Tyrosinkinase bcr/abl kann als die Ursache der unkontrollierten Zellteilung der CML-Zellen angesehen werden. Zur Behandlung von CML werden sogenannte Tyrosinkinase-Inhibitoren eingesetzt, die als spezifische Inhibitoren dieser Kinase wirken.

Im Rahmen der vorliegenden Erfindung wurde völlig überraschend festgestellt, dass Tyrosinkinase-Inhibitoren als Therapeutikum bei arterieller Hypertonie, bevorzugt bei essentieller arterieller Hypertonie, eingesetzt werden können.

Gegenstand der vorliegenden Erfindung ist daher die Verwendung eines Tyrosinkinase-Inhibitors zur Herstellung eines Arzneimittels zur Behandlung von Bluthochdruck.

Bevorzugt ist der Tyrosinkinase-Inhibitor Imatinib. Der Wirkstoff "Imatinib" ist derzeit unter der Bezeichnung Glivec® erhältlich. In den Handel kommen bspw. Hartgelatine Kapseln mit 100 mg Wirkstoff.

In bevorzuger Ausführungsform wird das Arzneimittel in einer Dosierung formuliert, die eine Konzentration zwischen 0,1 und 10 mg Tyrosinkinase-Inhibitor pro kg des zu behandelnden Patienten aufweist.

Bevorzugter wird aber eine pharmazeutische Formulierung, die eine Konzentration von 0,5 bis 5 mg Tyrosinkinase-Inhibitor pro kg Körpergewicht ergibt. Besonders bevorzugt ist eine pharmazeutische Formulierung, die eine Konzentration von 1,0 bis 3 mg Tyrosinkinase-Inhibitor pro kg Körpergewicht ergibt. Die fertigen Tabletten, Kapseln und ähnliches weisen daher bevorzugt einen Gehalt von nur 5, 10, 20 oder 25 mg Imatinib per Einheit auf.

### Beispiel

Eine Patientin litt seit Jahrzehnten unter einer schweren arteriellen Hypertonie. Trotz Einnahme von blutdrucksenkenden Mitteln aus verschiedenen Wirkstoffgruppen wies sie über Jahre hinweg Blutdruckwerte von bis zu über 200 mm Quecksilbersäule systolisch auf. Durch den jahrelangen hohen Blutdruck hatten sich bereits Folgeschäden an den Nieren, dem Herzen (Herzinfarkt) und ein Schlaganfall eingestellt. Der Blutdruck dieser Patientin wurde als medikamentös nicht ausreichend mit bekannten Medikamenten einstellbar angesehen.

Bei einer Überwachung des Blutdrucks über 24 Stunden wurde festgestellt, dass selbst bei maximaler blutdrucksenkender Therapie noch immer 94 % aller Messungen zu hohe Blutdruckwerte ergaben.

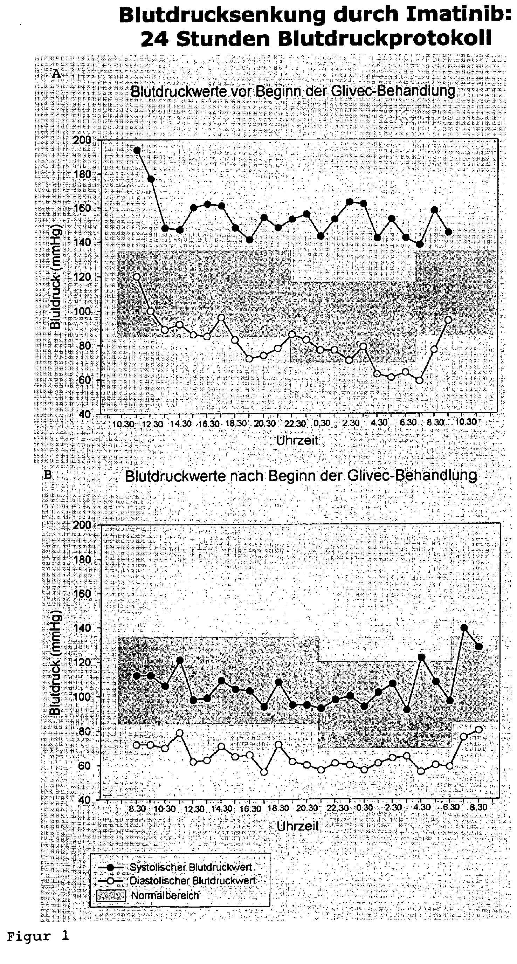

Die bei dem vorliegenden Beispiel erhaltenen Werte sind in der Figur 1 dargestellt.

Der Teil A der Figur 1 stellt die systolischen und diastolischen Blutdruckwerte ohne Imatinib-Behandlung (Glivec®) dar. Es ist klar ersichtlich, dass der Blutdruck der Patientin zu hohe Werte ergab.

Vor der Verabreichung von Imatinib lag der Blutdruck dieser Patientin trotz Einnahme von neun verschiedenen blutdrucksenkenden Medikamenten noch immer um 180/110 mm Quecksilbersäule.

Daraufhin wurde der Patientin eine Dosis Imatinib (400 mg) verabreicht. Schon nach wenigen Stunden sank der Blutdruck so dramatisch ab, dass sich bei der Patientin aufgrund der niedrigen Blutdruckwerte Schwindel einstellte.

Die Patientin wurde daraufhin mit einer geringeren Dosis Imatinib (100 mg pro Tag) so eingestellt, dass keine weiteren blutdrucksenkenden Mittel erforderlich waren. Das Blutdruckprotokoll zeigt aufgrund der Verabreichung des Tyrosinkinase-Inhibitors Imatinib durchwegs normale bis hypotone Blutdruckwerte.

Der untere Teil der Figur 1 (B) zeigt die Blutdruckwerte nach Verabreichung von Imatinib (Glivec®). Aufgrund der erfindungsgemäßen Verwendung konnte der Blutdruck auf gewünschte Werte gesenkt werden.

Ohne an eine Theorie gebunden sein zu wollen, könnte der blutdrucksenkende Wirkungsmechanismus von Tyrosinkinase-Inhibitoren über den PDGF-R (Platelet-Derived Growth Factor Receptor) vermittelt werden. So leiden PDGF-R knock-out Mäuse an einer absoluten Defizienz von Perizyten, einer speziellen Zellart, die eine wesentliche Strukturkomponente der Kapillaren darstellt. Ohne PDGF-R ist eine normale Ausbildung von Kapillaren nicht möglich und, wie das PDGF-R knock-out Mausmodell zeigt, kommt es zur Ausbildung von aneurysmatischen Aussackungen im Kapillarbett. Zusätzlich kann Imatinib den interstitiellen Gewebedruck in solidem Tumorgewebe verringern und diese drucksenkende Wirkung könnte durch Hemmung des PDGF-R vermittelt werden. Der PDGF-R könnte eine entscheidende Rolle für die anatomische Entwicklung und die Funktion der Mikrozirkulation im Gewebe spielen. Die Blutgefäße der Mikrozirkulation und ihr Funktionszustand sind, neben der Niere, die essentiellen Organkomponenten, die für die Regulation des systemischen Blutdrucks verantwortlich sind. Es wäre also möglich, dass durch die vorliegende Erfindung ein neues Behandlungsprinzip der arteriellen Hypertonie offenbart wird.

Die erfindungsgemäße Verwendung wird bevorzugt zur Behandlung von Patienten eingesetzt, bei denen eine ausreichende Reduzierung des Blutdruckes mit üblichen Antihypertensiva nicht erreicht werden kann. Dieses Patientenkollektiv kann dadurch charakterisiert werden, dass diese eine schwer einstellbare arterielle Hypertonie aufweisen.

Die erfindungsgemäß eingesetzten Tyrosinkinase-Inhibitoren können auch, zusammen mit anderen blutdrucksenkenden Mitteln, eingesetzt werden, die über andere Wirkmechanismen ihre therapeutische Wirkung entfalten.

## Patentansprüche

1. Verwendung eines Tyrosinkinase-Inhibitors zur Herstellung eines Arzneimittels zur Behandlung von Bluthochdruck.

2. Verwendung nach Anspruch 1, **dadurch gekennzeichnet, dass** der Tyrosinkinase-Inhibitor Imatinib ist.

3. Verwendung nach Anspruch 1 oder 2, **dadurch gekennzeichnet, dass** es sich bei dem Bluthochdruck um essentielle arterielle Hypertonie handelt.

4. Verwendung nach einem der vorhergehenden Ansprüche, **dadurch gekennzeichnet, dass** das Arzneimittel in einer Dosierung formuliert wird, die eine Konzentration zwischen 0,1 und 10 mg Tyrosinkinase-Inhibitor pro kg des zu behandelnden Patienten ergibt.

5. Verwendung nach Anspruch 4, **dadurch gekennzeichnet, dass** die pharmazeutische Formulierung eine Konzentration von 0,5 bis 5 mg Tyrosinkinase-Inhibitor pro kg Körpergewicht ergibt.

6. Verwendung nach Anspruch 4, **dadurch gekennzeichnet, dass** die pharmazeutische Formulierung eine Konzentration von 1,0 bis 3 mg Tyrosinkinase-Inhibitor pro kg Körpergewicht ergibt.
